# EUROPEAN PATENT SPECIFICATION

(11) **EP 3 538 062 B1**
(45) Date of publication and mention of the grant of the patent: **18.03.2026**
(21) Application number: 17807936.4
(22) Date of filing: 06.11.2017
(51) Int. Cl.: A61Q 7/00, A61Q 19/00, A61K 8/34, A61K 8/44, A61Q 5/00, A61Q 5/02, A61Q 5/12

(54) **ASSOCIATION CONSISTING OF INOSITOL AND ARGININE FOR MAINTAINING HOMEOSTASIS OF THE HAIR AND SCALP**
ZUSAMMENSETZUNG BESTEHEND AUS INOSITOL UND ARGININ ZUR AUFRECHTERHALTUNG DER HOMÖOSTASE VON HAAR UND KOPFHAUT
COMPOSITION À BASE D'INOSITOL ET D'ARGININE POUR LE MAINTIEN DE L'HOMÉOSTASIE DES CHEVEUX ET DU CUIR CHEVELU

(30) Priority: 08.11.2016 IT 201600112535
(43) Date of publication of application: 18.09.2019
(73) Proprietor: Unifarco S.p.A., 32035 Santa Giustina (BL) (IT)
(72) Inventor: BARATTO, Giovanni, 32035 Santa Giustina (BL) (IT)
(74) Representative: Perani & Partners S.p.A.
(86) International application number: PCT/IB2017/056916
(87) International publication number: WO 2018/087643

(56) References cited:
- WO-A1-2006/066323
- WO-A1-96/21421
- WO-A2-2007/113391
- CN-A- 101 683 344
- DE-A1- 102005 012 021
- FR-A1- 2 864 445
- US-A- 5 053 230
- DATABASE GNPD [online] 31 March 2016 (2016-03-31), "Hyaluronic Density Conditioner", XP002769052, retrieved from Mintel accession no. 3866053 Database accession no. 3866053

## Description

### FIELD OF THE INVENTION

The present invention relates to an association for topical use with osmoprotectant action as laid out in the appended claims.

### DESCRIPTION

### PRIOR ART

The native structure of a protein is essential in the biological field to the correct functioning of the metabolic processes of a living organism.

Environmental conditions can alter the stability of biomolecules, sensitive to changes in temperature, pressure and concentration of salts and other solutes.

Under physiological conditions, therefore, proteins require adaptation systems that enable to counterbalance any significant disruption of the cell thermodynamic balance, in order to prevent significant alterations in the secondary and tertiary structure. With the aim to adapt to environmental changes, nature has created physiological mechanisms of response, one of which involves the accumulation of small organic solutes, osmolytes, at the cellular level.

Naturally occurring in nature, osmolytes (osmoprotecants) are generally neutral at physiological pH and are stored in the intracellular environment at relatively high concentrations, exerting a thermodynamic stabilization action, without interfering with normal cellular functions.

In osmotic stress conditions, for example, living organisms increase the de novo synthesis of endogenous osmoprotectants at the cellular level, so as to restore the correct osmotic pressure values between the intra- and extracellular environment, as well as stabilize the tertiary structures of functional proteins.

Despite the chemical variety of osmoprotectants present in nature, in almost all living organisms we can recognize three major classes of endogenous osmolytes: some amino acid derivatives (taurine, betaine, etc.), polyols (sorbitol, glycerol, sucrose, trehalose, etc.) and methylamines (trimethylamine-N-oxide, choline-O-sulfate).

The physical chemical profile of each of these solutes is different from that of other classes of osmolytes, making it difficult to understand how, and to what extent, the properties of the ones can alter the function of the others in the displaying of the osmoprotectant effect of maintaining homeostasis, as well as the mechanism of action put in place for the thermodynamic stabilization of protein folding.

Among the different theories proposed in the literature to justify the complex osmoprotectant effect of compatible solutes, the most accredited one provides that the osmolyte-biomolecule interaction is the result of two stabilizing effects, one addressed directly to the macromolecule, the other indirect, mediated through the solvent.

The direct effect that the osmoprotectant exerts on the protein chain is called osmophobic effect and provides thart osmolyes are preferentially excluded from the protein surface, favoring the folding of the biomolecule using a thermodynamically unfavorable interaction with the main chain of the protein itself.

In addition to this first effect, a second one is added, directed at strengthening the local order of the microscopic structure of the solvent, by the formation of a network of hydrogen bonds between compatible solute and of the surrounding water molecules. The solvent thus structured is less prone to interact with the hydrophobic residues of the protein, which are folded to form the core of the three-dimensional peptide structure and which thus ensure the formation of a thermodynamically stable tertiary structure.

The moisturizing and antioxidant properties of naturally occurring osmolytes are already known in the prior art, which lists various osmolyte-based formulations directed to the well-being and beauty of the skin, for the mitigation of the early signs of aging.

The European patent application EP2695600 (A1) by the same Applicant describes a cosmetic composition with osmoprotectant activity, comprising ectoine and trehalose for use in the treatment of skin dryness.

DE102005012021A1 discloses a formulation, preferably for preventing care, treating scalp and hair, preventing the hair loss and/or promoting the hair growth, comprises 0.001-6 wt.% of an inositol, 1x10->3>-1 wt.% of a milk protein, 1x10->3>-2 wt.% of a sulfur containing amino acid, 1x10->3>-1 wt.% of a glycoprotein, 0-75 wt.% of a denatured alcohol, 0-10 wt.% of an isopropanol, an acid or a base for adjustment of pH value of 6-7.5, and water made up to 100 wt.%.

WO2007113391describesto use of inositol for enhancing and/or accelerating swelling of a thickener into an aqueous solution. In addition to enhancing and/or accelerating swelling of a thickener compound, inositol also increases the thickening efficiency of the compound in a chemical composition.

WO2006066323A1 discloses a formulation for trichological treatment of a patient including, in a dosage in the range of 3 g to 8 g: phytosterols in the range of 200 mg to 2500 mg; the quantitative equivalent of Polygonum multiflorum root 4:1 in 70% EW in the range of 600 mg to 2600 mg; methylsulfonylmethane in the range of 500 mg to 2000 mg; and bovine colostrum in the range of 500 m to 2000 mg.

Database GNPD Mintel 31 March 2017 Accession no. 3866053 "Hyaluronic density conditioner discloses a hyaluronic acid based composition containing among a plethora of components also inositol and arginine in unspecified amount for increasing visible hair density, fullness and softness.

### SUMMARY OF THE INVENTION

The Applicant has now found that inositol and arginine, when combined together in specific molar ratios, as laid out in the appended claims, exhibit a remarkable effect, greater than the effect exerted by the individual active ingredients or combination of alternative solutes.

The present invention therefore relates to cosmetic compositions, medical devices comprising the combination of inositol/arginine for use in treatments aimed to the improvement of the scalp and of the hair and for the maintenance of the homeostasis thereof.

### DESCRIPTION OF THE FIGURES

Figure 1: Trend of the oxygen-oxygen radial distribution function of water in the absence (wat) and in the presence of single and mixed active ingredients: arginine (arg); myo-inositol (myo); taurine (tau); myo-inositol/arginine mixture 1:1 (mol/mol) (myo_arg_1_1); myo-inositol/arginine mixture 2:1 (mol/mol) (myo_arg_2_1); myo-inositol/taurine mixture 1:1 (mol/mol) (myo _arg_1_1); myo-inositol/taurine mixture 2:1 (mol/mol) (myo_arg_2_1); myo-inositol/arginine/taurine mixture 2:1:1 (mol/mol) (mix_ter_2_1_1).
Figure 2: Detail of the first peak in figure 1 of the oxygen-oxygen radial distribution function of water.
Figure 3: Self-diffusion coefficient of water in the absence (wat) and in the presence of the individual active ingredients and in a mixture in figure 1.
Figure 4: Solvent-accessible surface area (SASA) normalized for myo-inositol, in the various solutions as a function of the duration of the simulation.
Figure 5: Structure representative of the distribution of a 1 M solution of arginine around keratin. The solvent (water) is omitted for clarity.
Figure 6: Structure representative of the distribution of a 1 M solution of myo-inositol around keratin. The solvent (water) is omitted for clarity.
Figure 7: Trend of the preferential coefficient of arginine as a function of the distance from the protein.
Figure 8: Trend of the preferential coefficient of myo-inositol as a function of the distance from the protein.
Figure 9: Trend of the preferential coefficients of myo-inositol and arginine in a 1:1 mixture (mol/mol) as a function of the distance from the protein.
Figure 10: Structure representative of the distribution of myo-inositol and arginine in a 1:1 mixture around keratin. Arginine is in light gray, myo-inositol in dark gray. The solvent (water) is omitted for clarity.
Figure 11: Trend of the preferential coefficients of myo-inositol and arginine in a 1:2 mixture as a function of the distance from the protein.
Figure 12: Structure representative of the distribution of myo-inositol and arginine in a 1:2 mixture around keratin. Arginine is in light gray, myo-inositol in dark gray. The solvent (water) is omitted for clarity.
Figure 13: Trend of the preferential coefficients of myo-inositol and arginine in a 2:1 mixture as a function of the distance from the protein.
Figure 14: Structure representative of the distribution of myo-inositol and arginine in a 2:1 mixture around keratin. Arginine is in light gray, myo-inositol in dark gray. The solvent (water) is omitted for clarity.
Figure 15: Content of ATP (nM) after 24H of treatment; NC = Negative Control.
Figure 16: Content of ATP (nM) after 120H of treatment; NC = Negative Control.
Figure 17: Morphological evaluation of MTs using H&E staining.
Figure 18: Expression of CK6, revealed for each sample in duplicate using immuno-staining of the protein with a specific antibody, labeled with DAB staining; 40X magnification; NC = negative control.
Figure 19: Duplicate evaluation of COLIV by immunofluorescence (black and white); NC = negative control, Cyclosporin A; Inositol; Inositol + Arginine.

### DETAILED DESCRIPTION OF THE INVENTION

The present invention relates to an association of the active ingredients with osmoprotectant action inositol and arginine, wherein the molar ratio inositol:arginine is 1:1.

In the present invention, association refers to the set of two or more active ingredients, considered in isolation with respect to the pharmaceutical form in which they are delivered, able to exhibit a specific biological effect in vitro and in vivo.

Inositol is an important osmolyte synthesized by the human body, which protects the cells from osmotic stress, through the mechanisms described above in the prior art and common to several endogenous osmolytes.

Inositol exists in the form of nine different isomers: myo-, cis-, allo-, epi-, muco, neo-, scyllo-, and optical isomers D and L-chiro inositol.

Of all, the most common in nature is myo-inositol, the isomer cis-1, 2, 3, 5-trans-4, 6 of inositol.

Unlike inositol, arginine is not generally considered an osmoprotectant (Amino Acids, Peptides and Proteins in Organic Chemistry, Analysis and Functions of Amino acids and Peptides; John Wiley & Sons, View on Google Scholar), as it is characterized by chemical, physical and biological properties that do not make it conform to the profile of conventional osmolytes.

Arginine in fact, at physiological pH, has a net positive charge at the level of the guanidine group which makes it little adapted to pass through the plasma membranes, semi-permeable to small, uncharged lipophilic solutes.

Said amino acid is also known in the literature to be incompatible with some biological processes, sometimes acting as denaturant and protein destabilizer, thereby limiting the expansion of its applications (Water and Life: Comparative Analysis of Water Relationships at the Organismic, Cellular, and Molecular Levels; edited by George N. Somero, Charles B. Osmond, Carla L. Bolis; Chemical Assistance in Refolding of Bacterial Inclusion Bodies; Mona Alibolandi and Hasan Mirzahoseini; Medical Biotechnology Research Center, Pasteur Institute of Iran, Tehran 13169 43551, Iran; P. H. Yancey, M. E. Clark, S. C. Hand, R. D. Bowlus, and G. N. Somero, "Living with water stress: evolution of osmolyte systems," Science, vol. 217, no. 4566, pp. 1214-1222, 1982. View at Google Scholar).

While arginine is characterized by apparently unfavorable properties as regards osmoprotection, the Applicant has found that the combination of inositol/arginine in a molar ratio of 1:1 allows obtaining an association of active ingredients with remarkable osmoprotectant properties, stabilizing against keratin.

Said properties also have proved to be superior to those exhibited by the individual components inositol/arginine and better than those obtained by the association of two classic osmolytes.

The osmoprotectant effectiveness exhibited by the association of the present invention has been interpreted by the Applicant by means of molecular dynamics studies and simulations of keratin in silico models, reported in the experimental section of the present patent application, as well as experimentally demonstrated by in vitro studies on micro-tissutal cell cultures (MTs).

In the light of the results obtained by computational test, it is believed that the combination of organic solutes in review can help to increase the availability of inositol in solution, reducing the aggregation between molecules of inositol itself.

Referring to the experimental section of the present patent application for a more comprehensive explanation of the molecular mechanisms involved in the mixture of osmoprotectants according to the invention, the simulations in solution of myo-inositol and L-arginine in salt form suggest that the cyclic polyol exhibits the greatest effect of water structuring and arginine exhibits the greatest effect of reducing the coefficient of self-diffusion of the solvent.

The two parameters described above, involved in the protein folding and stabilizing the tertiary structure of the protein, however, are affected by the tendency of inositol to aggregate in solution and by the denaturant behavior that arginine exhibits against biological macromolecules.

The combination of inositol and arginine according to the association of the present invention allows mitigating the above undesirable behavior, generating an osmolytic mixture with remarkable osmoprotectant properties, which simultaneously preserves the peculiar behavior of the two substances and maximizes the possibility of mutual influence.

The inositol included in the association of the present invention is preferably myo-inositol.

For the purposes of the present invention, arginine is the 2-amino-5-guanidilpentanoic acid and the biologically acceptable salts of said compound.

More preferably, arginine is the levorotatory form of the amino acid (L-arginine).

In a preferred embodiment, arginine is in the form of cationic salt, wherein the anionic counterion is preferably selected from hydrochloride, sulfate, citrate, carbonate, acetate, tartrate, phosphate, and even more preferably is hydrochloride.

By biologically acceptable salt is meant a salt suitable for medical/cosmetic use that does not induce toxicity, irritation or allergy reactions when in contact with human and animal living tissues and which has, in terms of biological activity, a reasonable risk/benefit ratio.

The association according to the present invention is used in the maintenance of homeostasis of the scalp and the hair, where homeostasis refers to the ability of an organism, and constituent units thereof, to maintain, in physiological conditions, the internal physical-chemical conditions constant also when the external environmental conditions change.

The association for the use of the present invention is administered in the form of a composition in combination with suitable excipients and/or diluents, where suitable excipients or diluents are all those components that can make the formulation technologically suitable for its administration.

Another object of the present invention is the use of the osmoprotectant composition described in the present patent application in the beauty treatment of the scalp.

In the context of the present invention, beauty treatment of the scalp and the hair means the treatment of skin and structural imperfections that can alter the beauty and functionality of the skin and the hair.

Among the most common skin problems that can afflict the scalp are skin dehydration and dryness, damage from oxidative stress, irritation, local itching, dandruff, excess sebum, etc.

Among the most common structural imperfections that can afflict the hair are damage from oxidative stress, fall, rupture of the stem, loss of volume and shine, premature aging of the hair (white hair), etc.

The scalp is an ultra-specialized area of the skin where the hair is implanted which, like the other hairs, originate from a hair follicle, called bulb. The scalp being constituted by skin as any other areas of the body, is subject to problems such as alterations, abnormalities, disorders or diseases and therefore requires a dedicated hygiene and care, which enable the maintenance of its functionality and well-being.

For the purposes of the present invention, skin dryness or asteatosis means a problem of the scalp, resulting from lipid deficiency and which is often associated to a situation of accentuated dehydration. The skin, dried and dehydrated, is fragile and brittle and tends to flake.

The reduced hydrolipidic content of the skin causes dandruff, dry hair and itch to the head. Using hairdryer with too much hot air dehydrates the keratin and it makes it brittle and fragile. The use of shampoos with aggressive surfactants, alcoholic lotions with dehydrating effect, reduced nutritional intake of vitamins and minerals, stress, pollution can cause dehydration of the skin.

For the purposes of the present invention, damage from oxidative stress are the micro and macroscopic functional alterations of living organisms, induced at the cellular level by a pathological condition (oxidative stress) caused by the rupture of the physiological balance between the production and elimination of oxidizing chemical species, by the physiological defense systems.

In the tricologic field, one of the first effects of oxidative stress is weakening and graying of the hair, caused by damage to melanocytes made by free radicals. The effects of oxidation are not limited to depigmentation of the hair, but can also damage the hair bulb cells, inducing the birth of a fragile hair tending to fall.

Another object of the present invention relates to the formulation of the composition of the present invention in suitable cosmetic products, intended for the uses mentioned above. Said cosmetic products (leave on and rinse off) are preferably in the form of shampoo, oil, conditioner, gel, lotion, foam, cream, mask, tonic, butters and alcoholic solutions.

A further object of the present invention are medical devices comprising the composition object of the present application, preferably in the form of shampoo, oil, conditioner, gel, lotion, foam, cream, mask, tonic, butters and alcoholic solutions.

### EXPERIMENTAL STUDIES

### 1. Studies in silico

### 1.1. Methodology

The simulated water-active systems consist of cubic boxes of water (solvation boxes), which are described using the TIP4P model and having a side of 50 Å, wherein the osmoprotectants were inserted at random positions. In the case of arginine (positively charged), the electroneutrality of the system was maintained by adding a suitable number of chloride ions.

The initial structure of the keratin pattern was obtained from the PDB database (code 3TNU). Also in this case, the protein was inserted at the center of a virtual box of size 170 x 60 x 60 Å (612000 Å³ volume), in which a quantity of active molecules necessary to achieve the desired concentration (1 M total) was added. The active ingredients were placed in random positions inside the solvation box and then the system was solvated with water, described by the TIP4P model. The electroneutrality of the system containing the protein was ensured by adding an adequate number of chloride ions, according to the amount of any arginine present.

The simulations were performed using the software package GROMACS 4.5.3. The system thus prepared was subjected to 10,000 steps of geometry optimization alternatively using the methods of the steepest descent (SD) and of the conjugate gradient (CG), in particular 1 SD step every 10 GC steps. The system was then balanced to 100 ps (picoseconds), at constant volume and temperature, followed by an additional 100 ps at constant pressure and temperature. During equilibration, the C_{α} atoms of the protein were fixated to their original position with a harmonious bond. The equilibration was followed by a production phase with a duration of 20 ns, in conditions of constant pressure and temperature, in which the bonds on C_{α} were removed. The temperature of the system was maintained at the reference value (310 K) by rescaling according to a suitable algorithm the velocities of the constituent particles of the system, while pressure was maintained at an average value of 1 bar isotropically coupling the system to a baric bath via the Berendsen algorithm. The bond distances between all the atoms were maintained at their equilibrium value using the algorithm LINKS, which made it possible to use an integration step of 2 fs (femtoseconds).

All simulations were carried out using periodic boundary conditions, the electrostatic interactions were assessed by using the Particle Mesh Ewald method, while a cut-off of 14 Å was applied for the interactions of Van der Waals.

To describe the protein, the AMBER99SBildn force field was used, while osmoprotectants were described by the GAFF force field. The analysis of the simulations were carried out with dedicated modules of the GROMACS package and programs implemented at the research group.

### 1.2. Simulations of water-active solutions

In order to assess the behavior of inositol and arginine in salt form in an aqueous solution, the Applicant conducted molecular dynamics simulations of each of the two molecules in water at a concentration of 1 M.

Said molecular dynamics simulations were conducted by selecting specifically myo-inositol and L-arginine, which represent the isomers most commonly found in vivo of the two molecules in question.

The electroneutrality of the L-arginine solution was ensured by adding a chloride ion for every molecule of amino acid.

The first visual analysis of the trajectories immediately revealed a qualitative difference between the salified amino acid and myo-inositol. While arginine during the simulation remains uniformly distributed in the volume of the solvation box, myo-inositol tends to quickly form aggregates.

This difference is attributable to the structure and properties of the different molecules. The salt of L-arginine, due to its ionized structure, has no tendency to aggregate and tends to be well solvated in solution.

Myo-inositol on the other hand, neutral and rich in hydroxyl groups, tends to form a network of interactions of hydrogen bonds, both with the solvent molecules and with other molecules of myo-inositol. In addition, the hydrophobic and Van der Waals interactions between the aliphatic rings of myo-inositol help to make favorable, from an entropy and energy viewpoint, the formation of aggregates of myo-inositol.

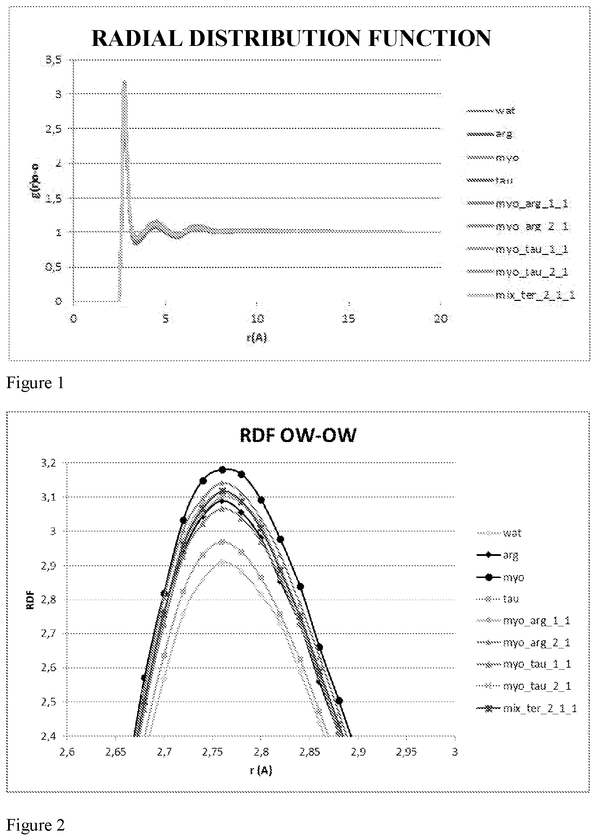

Subsequently, the effects exerted by the two molecules on the local structure of water were analyzed (the results are shown in figures 1 and 2).

By analyzing the trajectories, for each solution, the radial distribution function between the water oxygens was obained (g(r) ₒ₋ₒ hereinafter), the trend whereof provides information about the microscopic structure and the water order level.

Figure 1 shows how the g(r) ₒ₋ₒ has in all cases a main peak around 2.8 Å, corresponding to the first solvation shell of the water molecule, i.e. the hydrogen bonds that it forms with its first neighboring molecules, giving the liquid a locally ordered structure.

A secondary peak is localized around 4.5 Å and is due to the second solvation shell, while a third peak barely perceptible is attributable to a third solvation shell. Beyond the threshold of 10 Å, the g(r) ₒ₋ₒ converges correctly to the unit value.

In Figure 2, which shows the enlarged detail of the first peak of solvation, it is observed that the presence of one or more osmoprotectants increases the maximum peak value without changing its position, indicating that the osmoprotectants strengthen the local order in the microscopic structure of water.

In particular, it should be noted (fig. 2) that the maximum increase in the first peak of g(r) ₒ₋ₒ, when compared with the pure water reference, is observed for the 1M solution of myo-inositol, while the second highest peak among the individual active solutions is observed in the case of 1M arginine solution.

As regards the mixtures, instead, it should be noted that the increase of the peak of g(r) ₒ₋ₒ compared to the pure water reference is intermediate compared to that of the individual components, with a weighted average with respect to their mutual relationship in which the compatible solutes were combined.

The mixtures richest in myo-inositol (myo_arg 2:1; myo_tau 2:1) therefore show, the other cosolvent being equal, a peak more pronounced than those in which the ratio is 1:1 (myo_arg 1:1; myo_tau 1:1).

The mixtures myo-inositol-L-arginine hydrochloride show a greater water structuring with respect to combinations containing myo-inositol - taurine, showing that the mixture of two classic osmoprotectants is not equally effective in terms of osmoprotection as the combination of solutes of the present invention.

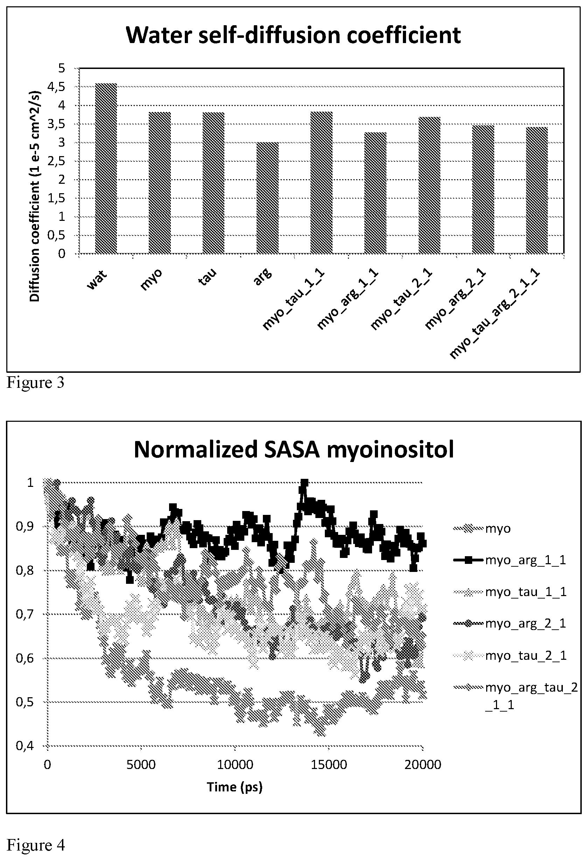

The coefficient of water diffusion in the various solutions was then calculated, comparing it with that of pure water. Figure 3 shows how the 1M solution of L-arginine exerts the most efficient decrease in the self-diffusion coefficient of all solutions. Also in this case, the values observed for the mixtures myo-inositol-arginine and L-myo-inositol-taurine are essentially a weighted average of those of the individual cosolvents.

Finally, a particular aspect of the mixtures was investigated, linked to the effects of the presence of a second or possibly a third cosolvent on the behavior of myo-inositol.

As mentioned above, the peculiar characteristic of myo-inositol compared to other osmoprotectants is the tendency to form aggregates. This trend is highlighted in figure 4, where the trend over time of the normalized solvent-accessible surface area (SASA) of myo-inositol in solution is shown.

A decrease in SASA over time indicates the formation of compact aggregates of myo-inositol. In Figure 4 it can be seen that the SASA of myo-inositol, when present alone in solution, is reduced by about 50% during the dynamics.

The simultaneous presence of L-arginine hydrochloride and myo-inositol in 1:1 ratio has been shown to limit the reduction of SASA to less than 20%.

The finding suggests the formation of less compact aggregates of myo-inositol, mixed with water molecules and L-arginine.

This greater homogeneity of the solution can be seen as a favorable element for the bioavailability of its components.

The combination of myo-inositol with another osmoprotectant, such as taurine, in a proportion of 1:1 has a much more limited effect than elicited by the combination according to the present invention.

The mixture with three components myo-inositol/taurine/L-arginine in a ratio of 2:1: 1 has been tested without success, since the simultaneous presence of two osmoprotectants proved to be less effective than L-arginine alone in reducing the aggregation in solution of myo-inositol.

### 1.3. Simulations of keratin with myo-inositol and L-arginine

In the second phase of the research we studied the interaction of a keratin model with the myo-inositol and L-arginine hydrochloride solution analyzed in the previous phase of the study.

Keratin is a fibrous protein consisting of a large number of helixes interconnected by disulfide bridges whose mutual winding creates the hair fibers. Since it was not possible to simulate an entire fiber, it was decided to use as a model a fundamental unit of keratin, consisting of two keratin α-helixes, joined to form a dimer.

The chosen protein model was simulated in a 1M solution of each of the two components considered. Also in this case, they showed a diametrically opposite behavior.

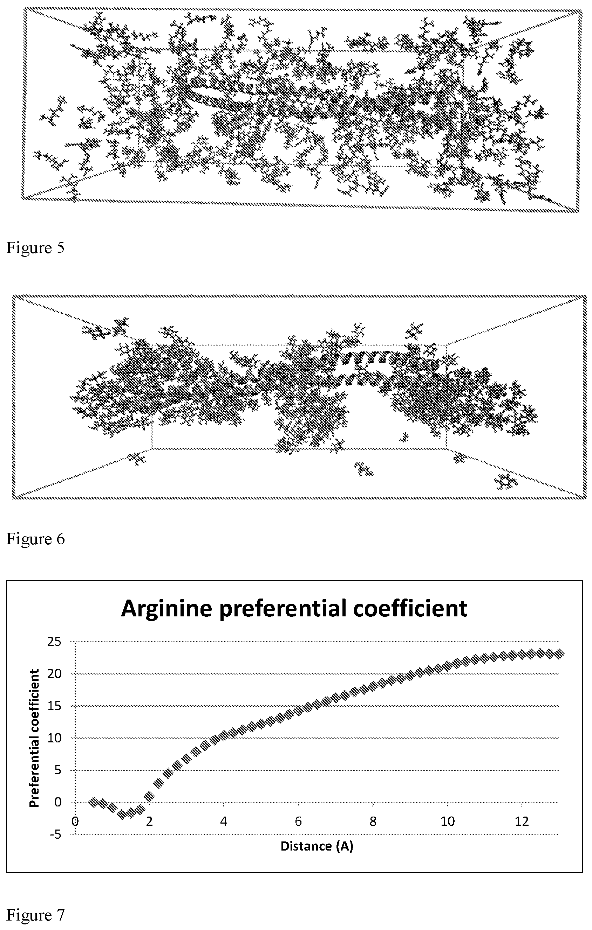

As can be seen in figures 5 and 6, while L-arginine hydrochloride appears uniformly distributed in the solvation box (schematically represented by the geometric shape of the parallelepiped), myo-inositol thickens clearly around the protein, showing both the already mentioned tendency to aggregation, and a preferential distribution in the immediate protein neighbourhood.

Subsequently, the behavior of L-arginine hydrochloride and myo-inositol was analyzed in a more quantitative manner, by calculating the preferential coefficient with respect to the protein for each of them, where preferential coefficient is a measure of the thickening or rarefaction of the organic molecule as a function of the distance from the protein surface.

A positive preferential coefficient indicates a thickening of the active ingredient with respect to the bulk of the solution, while a negative coefficient indicates a rarefaction.

In Figure 7 we can observe that the preferential coefficient of L-arginine hydrochloride has the trend of that of a classic osmoprotectant, being negative at low distances from the protein (osmophobic effect index).

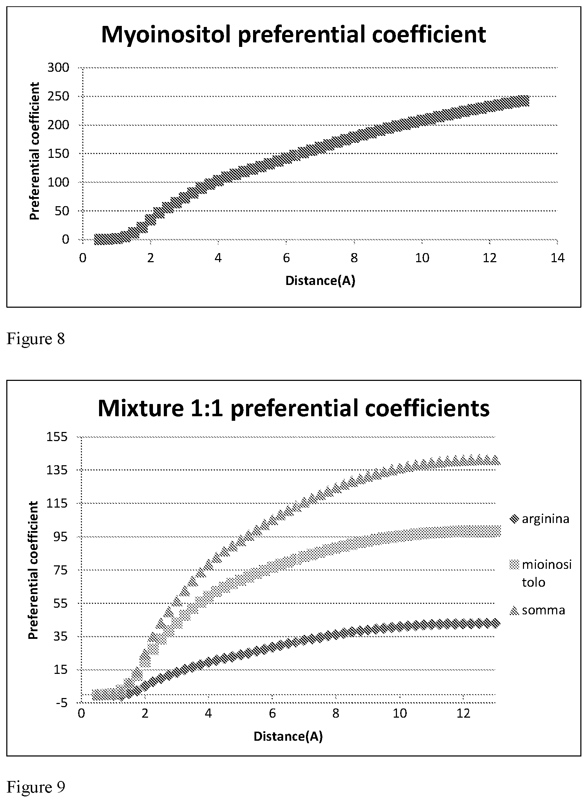

The preferential coefficient of myo-inositol is on the contrary positive (fig. 8) even at low distances, assuming values generally higher, indicating a considerable thickening of myo-inositol around the protein surface.

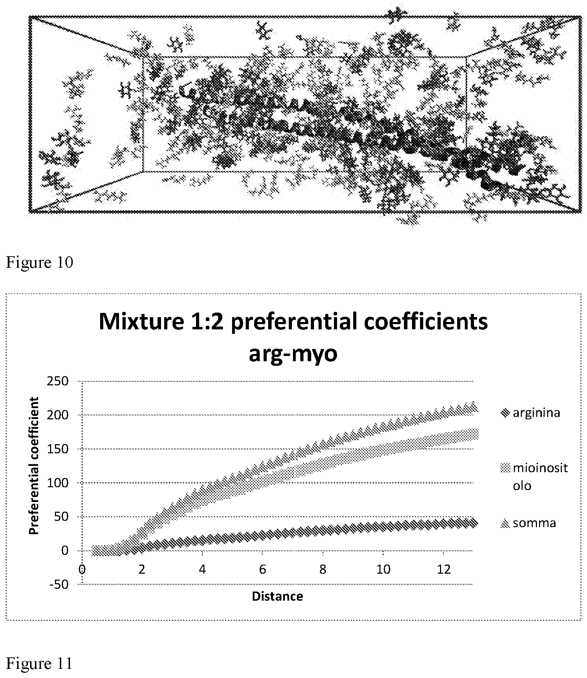

When the two molecules are present simultaneously in the ratio of 1:1 (figures 9 and 10), each of them retains its basic behavior, with myo-inositol thickened around keratin and L-arginine hydrochloride more evenly distributed in the solvation box.

The tendency of myo-inositol to self-aggregation is however limited by the presence of L-arginine hydrochloride.

This creates what may figuratively be called a double layer of osmoprotecants around keratin, with myo-inositol closest to the protein and L-arginine more distant.

In L-arginine/myo-inositol solutions in the ratios of 1:2 and 2:1, we see a prevalence of the most abundant co-solvent behavior.

In particular, in the 1:2 solution, L-arginine hydrochloride, present in defect, is unable to significantly reduce the aggregation of myo-inositol, which is preferentially thickened on keratin.

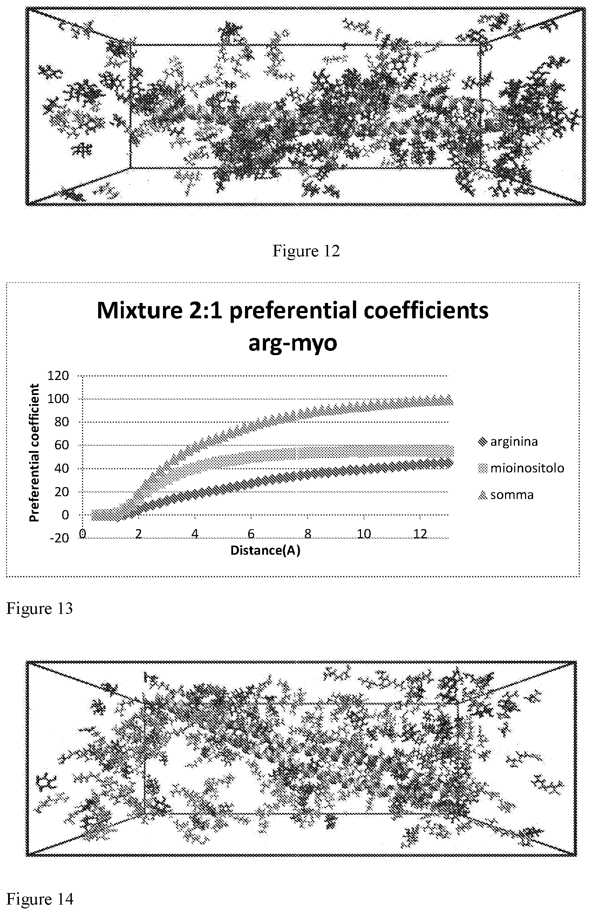

When instead L-arginine hydrochloride is present in excess, its action destroys the double-layer structure of osmoprotectants and myo-inositol is widely dispersed in the solvation box, being divided to a much lesser extent in the vicinity of keratin (fig. 11-14).

### 2. In vitro studies

### 2.1 Introduction and scope of the study

The Microtissue (MTs) technology represents a new in vitro cell culture method in which the cells, rather than adhering to a substrate, forming a two-dimensional monolayer, clump together in small volumes of the culture medium, forming a three-dimensional structure in which spatial and molecular interactions cell-cell and cell matrix are better representative of the interactions characterizing the living tissue in nature. The cells cultured in accordance with the MTs technology can grow and organize themselves according to their natural physiology, reproducing in vitro (at least partially) the typical morphology and functionality of their tissues of origin. For these reasons, the in vitro test conducted in MTs are potentially better predictive than in vitro tests carried out in two-dimensional culture. The MTs technology allows, by means of the action of the force of gravity, the formation of microtissue aggregates at the liquid-air interface of culture medium drops. Avoiding contact with any support materials, cells recreate the tissue in a natural way and the resulting microtissue aggregate constitutes a relevant in vitro system for research and pre-clinical applications.

### 2.2. Application of MTs technology to the hair follicle

The hair follicle (HF) is a "mini-organ" capable of self-regenerating and that continues to alternate phases of growth and regression, according to a precise sequence. The renewal process begins in the hair bulb at the level of the dermal papilla (DP), a spherical core of mesenchymal cells, subsequently affecting the epithelial cells of the follicle that surround and enclose the dermal papilla. The molecular mechanism underlying the growth cycle of the hair follicle is very complex and still not fully elucidated. However, it is known that the epithelial-mesenchymal interactions, and in particular the mutual communication between the dermal papilla fibroblasts (DPF) and the outer root sheath keratinocytes (ORSK) play a decisive role in the hair growth/regeneration cycle.

The availability of a co-culture model of DPF-ORSK allows investigating the mechanisms that regulate the growth cycle of the hair follicle and studying the effects of active compounds in pre-clinical research. As reported in the study of Higgins et al. (2013, PNAS), the gene expression profile of dermal papilla cells is modified deeply when the same are grown with monolayer growing techniques, but may be partially restored when the dermal papilla cells are cultured in 3D spheroids (MTs). What has just been said draws attention to the fact that the spatial distribution of the cells, and thus their possibility of communication, is essential to reproduce a realistic tissue model and the use of co-spheroidal cultures of DPF and ORSK is therefore the optimal method to reproduce a "proto-follicle," as it does not include an artificial substrate.

The tissue model of co-spheroidal culture DPF-ORSK has therefore been used in the in vitro studies presented below for the reproduction of a "proto-follicle" in the evaluation of the biological effects of arginine and inositol and mixtures thereof concerning:
- Increased hair renewal and well-being
- Improvement of the structure, morphology and metabolism of the hair follicle
- Improvement of the structure and strength of the hair.

### 2.3. Methodology

The reconstituted proto-follicle was obtained by using fibroblast cells and human keratinocytes using the Gravity^{PLUS™} method, a system developed by InSphero (InSphero AG, Zurich, Switzerland), which allows the formation of spheroids or micro-tissues of homogeneous size using the hanging drops technology. The micro-tissues were transferred to Gravity^{TRAP} plates for treatment and control of culture conditions: 37 °C incubator, 5% CO₂, suitably saturated atmosphere of the culture medium.

The batch of fibroblasts and keratinocytes were tested for the absence of HIV, hepatitis B, hepatitis C, mycoplasma. The maintenance medium was tested for sterility.

The effect of the following active compositions was evaluated on the micro-tissue cultures described above:
- Inositol 0.5%
- Inositol 0.25% + L-arginine 0.25%

The results obtained with the above compositions were compared with the results obtained by:
- Negative control (untreated)
- Positive control: Cyclosporin A 10 ng/mL, as a reference substance, known to be active in the stimulation of the anagen phase of the hair follicle cell proliferation in in vivo and ex vivo cell cultures (Havlicova et al. 2009).

In order to assess the biological effect of each of the above compositions on the micro-tissue cultures in question, the following parameters were measured:
- Quantification of ATP, as a marker of cell viability.
- Morphological evaluation during culture of the proto-follicle, by staining with hematoxylin-eosin (H&E).
- Immunohistochemical evaluation of Cytokeratin 6 (CK6), wherein CK6 is a keratin type II present in the skin and the hair follicle, which has an important role in the formation of the intermediate filament and is involved in tissue remodeling during stress phenomena.
   CK6 seems to be involved in the formation of the correct cellular architecture, suitable for migration and cell resistance (Wojcik 2001) and consequently responsible for the structure and strength of the skin and its appendages (Wong 2003).
- Semi-quantitative determination of type IV collagen (COLIV), where COLIV is the major component of the basal lamina and is part of the follicular-papillary dermal junction, but is also present in extracellular granular form within the follicular sheath, where it exerts an anchoring function during the active growth phase (anagen) of the hair follicle (Jahoda 1992).

In order to observe the short- and long-term effect of the compositions mentioned above, the evaluation of the above parameters was made at 24H (T2) and 120H (T6) after the beginning of therapy.

### 2.4. Results

### 2.4.1. ATP content

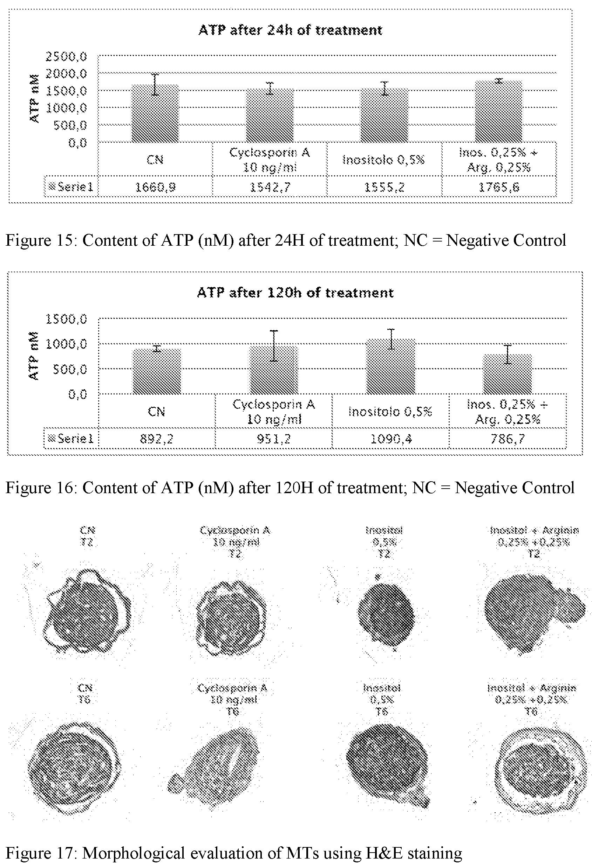

Figures 15 and 16 show the content of ATP at 24H and 120H after the beginning of treatment, in the samples treated with inositol 0.5% and inositol 0.25%/L-arginine 0.25% in comparison with the untreated negative control and the sample treated with cyclosporin A.

No significant alteration of the ATP content was recorded at 24H and 120H after the beginning of treatment, indicating that none of the compositions under examination caused substantial modifications of homeostasis and metabolic activity of the cultures.

### 2.4.2 Morphological evaluation during culture of the proto-follicle

Figure 17 shows the morphological qualitative evaluation of micro-tissues (MTs) by H&E staining.

As expected, the micro-tissue has a multi-layered structure with a dense core of dermopapillar fibroblasts surrounded by a stratification of keratinocytes. The keratinocytes appear perfectly layered and polarized around the dermal core, with some detectable differences:
- **Negative control:** The layers of keratinocytes at T6 appear thicker compared to the preliminary results at T2 and partially detached from the core of fibroblasts. This detachment is intrinsically linked to the fragility of the micro-tissue cultures (MTs).
- **Cyclosporin A:** at T6, the cell nuclei appear much denser in comparison with the surrounding cytoplasm, indicating a possible toxic effect of the compound, in this case used at the reference concentrations in the literature for the experimental conditions adopted.
- **Inositol 0.5%:** Both at T2 and at T6, the layers of keratinocytes surrounding the fibroblasts seem to be closely related to the dermal core and the keratinocyte cell layer has a more compact lamellar structure.
- **Inositol/Arginine 0.25%/0.25% mixture:** At T2, the morphology is not different from what was observed for the sample treated with Inositol; on the contrary, at T6 the morphology indicates an increase in the cell differentiation resulting in a partial detachment of the epithelial layers from the dermal core. In any case, the micro-tissues have a better organization of the lamellar structure and a thickness greater than the negative control, where the epithelial layers were thinner and less compact, characteristics indicating a greater tissue fragility.
In conclusion, the treatment with the active mixture comprising inositol and L-arginine seems to promote, at 120H from the beginning of therapy, a significant cell differentiation without inducing fragility and cell loss.

### 2.4.3. Immunohistochemical evaluation of cytokeratin 6 (CK6) of the hair follicle.

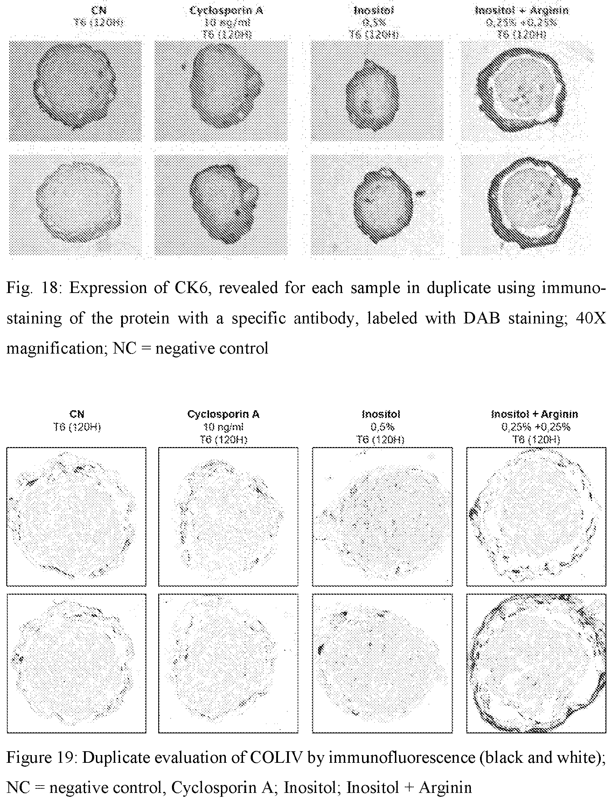

Figure 18 shows the immunohistochemical evaluation of CK6.

The follicular CK6 expression is different depending on the treatment to which the culture was subjected and in particular:
- **Cyclosporin A:** the layers of keratinocytes appear to be in good connection with the papillary core compared to the negative control, which shows instead a partial detachment in the outer layer. The results are consistent with the known properties of the molecule in the stimulation of growth of the hair follicle.
- **Inositol 0.5%:** The proto-hair structure and coloring are not different from those shown by the samples treated with Cyclosporin A, suggesting efficacy on cell growth of inositol 0.5%.
- **Inositol/Arginine 0.25%/0.25% mixture:** The samples treated with the above mixture show, compared to those treated with cyclosporin and with inositol alone, a specific effect at the level of the keratinocyte layer, which is more differentiated, thicker and more structured, with a consequent partial detachment of said layer from the dermal core. The most important result consists in the detection of a strong signal corresponding to CK6, which indicates an over-expression of the protein in comparison with the other samples. This result suggests the presence of a possible effect of the Inositol/Arginine 0.25%/0.25% mixture on the keratinocyte terminal differentiation. The strong signal relative to CK6, protein with a structural function, correspond in fact to a better organization of the cell layers responsible, in vivo, of an increase in the thickness and strength of the hair shaft.

### 2.4.4. Assessment of collagen IV in confocal microscopy.

Figure 19 shows the results of duplicate evaluation of COLIV by immunofluorescence. Table 1 shows the results, in duplicate, obtained from the semi-quantitative analysis of CN, inositol and Inositol/Arginine 0.25%/0.25% mixture: it is clear that the expression of COLIV is significantly increased following treatment with the above active mixture.

The signal emitted by type IV collagen in the sample treated with the Inositol/Arginine 0.25%/0.25% mixture is particularly strong in the vicinity of the edges of the dermal papilla, close to the layer of keratinocytes, indicating an increase in the production of the structural protein at the level of the basal lamina. The increase at the microscopic level of COLIV synthesis reflects at the macroscopic level an increase of the resistance and solidity of the hair follicle. In the samples treated with the Inositol/Arginine 0.25%/0.25% mixture, a certain variability of the COLIV signal was recorded, associated with the normal biological variability of the sample. In fact, also from the visual analysis of the image, the first replicate shows a weaker signal compared to the second replicate, in which the epithelial layer is very well marked.

Despite the variability recorded between replicates, the signal detected in the samples treated with the above active mixture is much stronger than that obtained in the negative control and in the sample treated with inositol.

| **CN (120 H)** | **Inositol [0.5%] (120H)** | **Inositol + Arginine [0.25% + 0.25%] (120 H)** |
|---|---|---|
| 198 | 69 | 288 |
| 129 | 94 | 729 |

### 3. Conclusions

The results achieved with the in vitro studies presented above can be summarized as follows:
None of the compositions tested induces a significant alteration of ATP levels on the metabolic balance of the proto-follicle which, in the absence of a stress stimulus, is able to maintain in culture the physiological conditions of homeostasis until the sixth day.

From a morphological point of view, both cyclosporin A (known for its effects on the hair growth cycle) and the inositol/arginine mixture have changed the overall morphology of the proto-papilla, which is more structured, compact and less fragile when compared with the untreated control.

In regard to the expression of specific proteins cytokeratin 6 and collagen IV, the inositol/arginine mixture exhibited a positive effect on their production at follicular level, better than that exhibited by inositol alone. In particular:
- The active mixture comprising inositol/arginine exhibits a synergistic effect on the expression of the CK6 protein, maintaining the morphology of the keratinocyte layer similar to that of the negative control. These results suggest that the mixture has an effect on cellular differentiation and on the production of follicular keratins, with the end result of an increase in the thickness and strength of the hair shaft.
- Regarding collagen IV, the samples treated with the inositol/arginine mixture showed a high expression of the protein at the level of keratinocyte layer, indicating an increase in the production of the structural protein at the level of the basal lamina, much greater than that recorded for inositol alone and for the negative control.
Considering the structural and anchoring functions performed by COLIV at the level of the hair follicle, the results recorded in vitro would result in vivo in an increase in stability and robustness of the hair follicle, suggesting the potential use of the inositol/arginine mixture in the treatment of hair and for the maintenance of its well-being.

### EXAMPLES

Below are some illustrative, non-limiting examples of cosmetic formulations containing the combination of arginine and inositol object of the invention.

### Example 1: conditioner

| | |
|---|---|
| Water | 75-100% |
| Emulsifying agents | 1 - 5% |
| Silicones | 1 - 5% |
| Emollients, wetting agents | 1 - 5% |
| Inositol | 0.1 - 1% |
| L-Arginine | 0.1 - 1% |
| Cationic surfactants ≥ C₁₂ | 0.1 - 1% |

## Claims

1. Association consisting of the active ingredients: inositol and arginine, combined in a molar ratio inositol: arginine of 1:1 for use in the maintenance of the homeostasis of scalp and hair.

2. Association for use according to claim 1 wherein inositol is myo-inositol.

3. Association for use according to any one of claims from 1 to 2, in the treatment of skin problems selected from the group consisting of asteatosis, irritation, local itching, dandruff and excess sebum.

4. Association for use according to any one of claims from 1 to 2, in the treatment of structural hair imperfections selected from the group consisting of fall, rupture of the stem.

5. Cosmetic, non-therapeutic use of an association consisting of the active ingredients: inositol and arginine, combined in a molar ratio inositol: arginine of 1:1 in the treatment of loss of volume and shine of the hair.

6. The cosmetic use according to claim 5 wherein inositol is myo-inositol

## Patentansprüche

1. Assoziation, bestehend aus den Wirkstoffen: Inositol und Arginin, kombiniert in einem Molverhältnis von Inositol:Arginin von 1:1, zur Verwendung bei der Aufrechterhaltung der Homöostase von Kopfhaut und Haar.

2. Assoziation zur Verwendung nach Anspruch 1, wobei das Inositol myo-Inositol ist.

3. Assoziation zur Verwendung nach einem der Ansprüche 1 bis 2, bei der Behandlung von Hautproblemen, ausgewählt aus der Gruppe bestehend aus Asteatose, Reizung, lokalem Juckreiz, Schuppen und überschüssigem Talg.

4. Assoziation zur Verwendung nach einem der Ansprüche 1 bis 2, bei der Behandlung von strukturellen Haarmängeln, ausgewählt aus der Gruppe bestehend aus Ausfall, Bruch des Schafts.

5. Kosmetische, nicht-therapeutische Verwendung einer Assoziation, bestehend aus den Wirkstoffen: Inositol und Arginin, kombiniert in einem Molverhältnis von Inositol:Arginin von 1:1, bei der Behandlung von Verlust von Volumen und Glanz des Haares.

6. Die kosmetische Verwendung nach Anspruch 5, wobei das Inositol myo-Inositol ist

## Revendications

1. Association consistant en les ingrédients actifs : inositol et arginine, combinés dans un rapport molaire inositol:arginine de 1:1, pour son utilisation dans le maintien de l'homéostasie du cuir chevelu et des cheveux.

2. Association pour son utilisation selon la revendication 1, dans laquelle l'inositol est le myo-inositol.

3. Association pour son utilisation selon l'une quelconque des revendications 1 à 2, dans le traitement de problèmes cutanés choisis dans le groupe consistant en l'astéatose, l'irritation, les démangeaisons locales, les pellicules et l'excès de sébum.

4. Association pour son utilisation selon l'une quelconque des revendications 1 à 2, dans le traitement d'imperfections structurelles des cheveux choisies dans le groupe consistant en la chute, la rupture de la tige.

5. Utilisation cosmétique, non thérapeutique d'une association consistant en les ingrédients actifs : inositol et arginine, combinés dans un rapport molaire inositol:arginine de 1:1, dans le traitement de la perte de volume et de brillance des cheveux.

6. Utilisation cosmétique selon la revendication 5, dans laquelle l'inositol est le myo-inositol.
